(19) Europäisches Patentamt
European Patent Office
Office européen des brevets

(11) Numéro de publication: **0 067 094**
**B1**

(12) # FASCICULE DE BREVET EUROPEEN

(45) Date de publication du fascicule du brevet: **02.10.85**

(21) Numéro de dépôt: **82400920.3**

(22) Date de dépôt: **18.05.82**

(51) Int. Cl.⁴: **C 07 D 209/48,** C 07 D 209/08, C 07 D 215/22, C 07 D 217/24, C 07 D 235/26, A 61 K 31/40, A 61 K 31/47, A 61 K 31/415

(54) **Dérivés hétérocycliques d'amidoximes, leur préparation et leur application en thérapeutique.**

(30) Priorité: **26.05.81 FR 8110428**

(43) Date de publication de la demande: **15.12.82 Bulletin 82/50**

(45) Mention de la délivrance du brevet: **02.10.85 Bulletin 85/40**

(84) Etats contractants désignés: **AT BE CH DE FR GB IT LI LU NL SE**

(56) Documents cités:
**DE - A - 2 252 805**
**DE - A - 2 412 519**
**DE - A - 2 724 368**
**FR - A - 2 150 590**
**FR - A - 2 392 967**
**US - A - 3 654 299**
**US - A - 3 658 832**
**US - A - 3 795 735**
**US - A - 4 048 176**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Obitz, Daniel, 2, Avenue François Molé, F-92160 Antony (FR)**

(74) Mandataire: **Thouret-Lemaitre, Elisabeth et al, Service Brevets - SYNTHELABO 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 067 094 B1

BUNDESDRUCKEREI BERLIN

**Description**

La présente invention concerne des dérivés hétérocycliques d'amidoximes, leur préparation et leur application en thérapeutique.

Des amidoximes ont déjà été décrits dans la littérature, par exemple dans le brevet français 7 401 286 et 7 810 109, par la Demanderesse dans son brevet français 7 811 024, et dans le prevet des Etats Unis d'Amerique 4 048 176.

Les compesés de l'invention répondent à la formule (I)

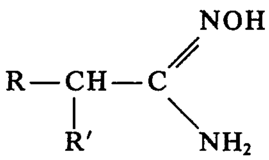

(I)

dans laquelle

R' représente un atome d'hydrogène ou le radical méthyle, et
R représente le radical oxo-1 1H-isoquinoléinyle-2 ou oxo-1 dihydro-1,3 2H-isoindolyle-2.

Les composés de l'invention peuvent être préparés selon le schéma réactionnel suivant:

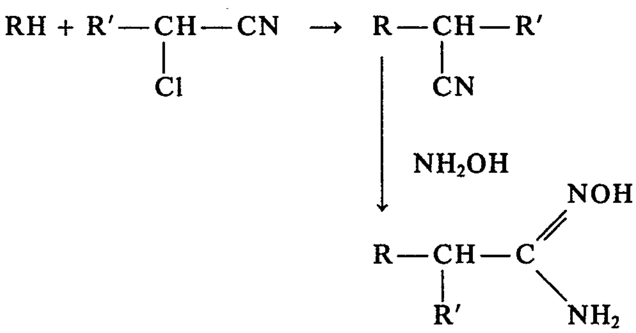


(I)

On fait réagir un composé RH avec un nitrile

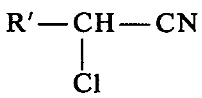

en présence d'hydrure de sodium dans un solvant tel que le diméthylformamide, puis on fait réagir le nitrile obtenu

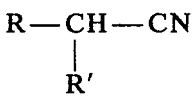

avec du chlorhydrate d'hydroxylamine dans un solvant tel qu'un alcool, en présence d'un équivalent de base, pour obtenir les composés (I).

Les composés de départ sont décrits dans la littérature.

L'exemple suivant illustre l'invention. Les analyses et les spectres IR et RMN ont confirmé la structure des composés.

Exemple

α-méthyl oxo-1 1H-isoquinoléine-2 acétamidoxime

1. α-méthyl oxo-1 1H-isoquinoléine-2-acétonitrile

Dans un ballon de 250 ml, on introduit 3,4 g (0,07 mole) d'hydrure de sodium à 50% que l'on met en suspension dans 50 $cm^3$ de DMF sec, on ajoute goutte à goutte, tout en agitant et sous azote, sec, une solution de 10 g (0,069 mole) d'isocarbostyrile dans 100 $cm^3$ de DMF sec.

Aprés une heure d'agitation à la température ambiante, la réaction est achevée.

On ajoute cette solution, goutte à goutte, à une solution de 7,16 g (0,08 mole) de chloro-2 propioni-

trile dans 100 $cm^3$ de DMF sec, refroidie á $-10°$ C.

Aprés une heure d'agitation à $-10°$ C, on laisse revenir le mélange réactionnel à la température ambiante et le laisse une nuit au repos (sous azote sec).

Le DMF est évaporé sous pression réduite, et le résidu est repris par de l'eau puis extrait par de l'éther. La phase organique, lavée à l'eau puis séchée sur $MgSO_4$, donne un résidu huileux qui est purifié sur colonne (Silice — $CHCl_3$ — $C_6H_6$ 5/5 et $CHCl_3$).

2. $\alpha$-méthyl oxo-1 1H-isoquinoléine-2 acétamidoxime

Dans un ballon de 500 ml, on introduit 9,8 g (0,049 mole) du nitrile précédent et 3,5 g (0,05 mole) de chlorhydrate d'hydroxylamine avec 150 $cm^3$ d'éthanol absolu. A cette suspension agitée sous azote sec, on ajoute goutte à goutte, une solution d'éthylate de sodium préparé par action de 1,15 g de sodium (0,05 mole) sur 50 $cm^3$ d'éthanol absolu. On chauffe ensuite 4 heures à la température du reflux. Après èvaporation de l'éthanol, on triture le résidu dans 100 $cm^3$ d'eau.

On lave le produit 3 fois à l'eau, puis le sèche. On le fait recristalliseur dans un mélange EtOH-MetOH 8/2.

Après filtration, lavage à l'éther et séchage à 60° C, sous vide, on obtient le produit.

F = 179—180° C

3. chlorhydrate du composé

A une suspension de 8 g (0,0346 mole) de base dans 100 $cm^3$ d'éthanol absolu, on ajoute un léger excès d'éther chlorhydrique. A la solution limpide obtenue, on ajoute 100 $cm^3$ d'éther et laisse cristalliser le produit, tout en agitant. Après filtration, lavage à l'éther et séchage à 60° C sous vide, on obtient le produit.

F = 182—185° C (déc.)

Tableau

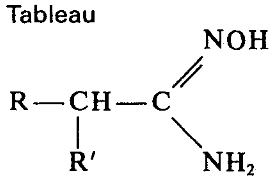

| Composé | R | R' | F (°C) |
|---|---|---|---|
| 1 | 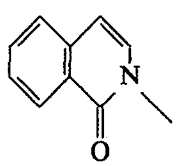 | $CH_3$ | base F = 179—180<br>HCl F = 182—185 |
| 2 | 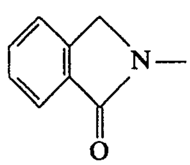 | H | base F = 210—212 (déc) |
| 3 | 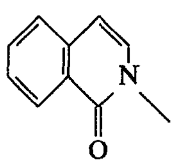 | H | base F = 224—226 (déc) |
| 4 | 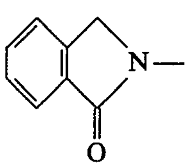 | $CH_3$ | base F = 175—176 (déc) |

Les composés de l'invention ont été soumis à des essais pharmacologiques.

La toxicité aigüe a été déterminée chez la souris après injection intrapéritonéale.

La DL 50 est de l'ordre de 200 à 1000 mg/kg i. p.

L'activité antidépressive a été déterminée par le test de l'antagonisme vis-à-vis de la ptose réserpinique (Gourat C. et al., J. Pharmacol. (Paris) 8, 333—350 (1977).

Les souris (mâles, CDI Charles River, France, 18—22 g) reçoivent simultanément les produits à étudier ou le solvant (voie i. p.), et la réserpine (4 mg/kg, voie s. c.).

Soixante minutes plus tard, le degré de ptose palpébrale est estimé au moyen d'une échelle de cotation (0 à 4), pour chaque souris.

A chaque dose, la moyenne de cotation et le pourcentage de variation par rapport au lot contrôle, sont calculés.

Pour chaque produit, la DA 50, ou dose qui diminue de 50% le score moyen de ptose par rapport aux contrôles, est déterminée graphiquement.

La DA 50 varie de 0,2 à 8 mg/kg par voie i. p.

Ces données montrent que les composés de l'invention peuvent être utilisés pour le traitement de diverses affections, en particulier pour le traitement de diverses affections du système nerveux central et pour le traitement de la dépression.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc. avec tout excipient approprié.

La posologie quotidienne peut aller de 5 à 500 mg.

**Revendications pour les Etats contractants: BE, CH, DE, FR, GB, It, LI, LU, NL, SE**

1. Dérivés hétérocycliques d'amidoximes, répondant à la formule (I)

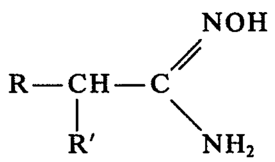

(I)

dans laquelle

R' représente un atome d'hydrogène ou le radical méthyle, et
R représente le radical oxo-1 1H-isoquinoléinyle-2 ou oxo-1 dihydro-1,3 2H-isoindolyle-2.

ainsi que leurs sels d'addition aux acides pharmaceutiquement acceptables, et les racémates et énantiomères des composés dans lesquels R' est $CH_3$.

2. Dérivés selon la revendication 1 dans lesquels R' est $CH_3$.
3. L'$\alpha$-méthyl oxo-1 1H-isoquinoléine-2-acétamidoxime.
4. L'oxo-1 1H-isoquinoléine-2 acétamidoxime.
5. Procédé de préparation des composés selon la revendication 1, procédé caractérisé en ce que l'on fait réagir un composé RH avec un nitrile

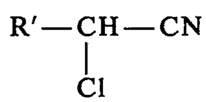

en présence d'hydrure de sodium puis on fait réagir le nitrile obtenu

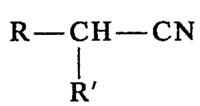

avec du chlorhydrate d'hydroxylamine.

6. Médicament caractérisé en ce qu'il contient un composé spécifié dans l'une quelconque des revendications 1 à 4.
7. Composition pharmaceutique caractérisée en ce qu'elle contient un composé spécifié dans l'une quelconque des revendications 1 à 4 en association avec tout exipient approprié.

**Revendication pour l'Etat contractant: AT**

Procédé de préparation de dérivés hétérocycliques d'amidoximes, répondant à la formule (I)

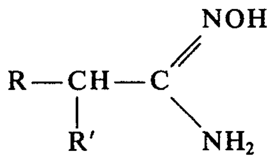

(I)

dans laquelle

R' représente un atome d'hydrogène ou le radical méthyle et,
R représente le radical
oxo-1 1H-isoquinoléinyle-2 ou
oxo-1 dihydro-1,3 2H-isoindolyle-2,

ainsi que de leurs sels d'addition aux acides pharmaceutiquement acceptables, et des racémates et énantiomères des composés dans lesquels R' est $CH_3$, procédé caractérisé en ce que l'on fait réagir un composé RH avec un nitrile

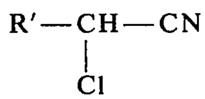

en présence d'une base telle que l'hydrure de sodium puis on fait réagir le nitrile obtenu

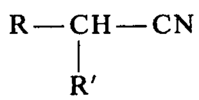

avec du chlorhydrate d'hydroxylamine.

**Patentansprüche für die Vertragsstaaten: BE, CH, DE, FR, GB, IT, LI, LU, NL, SE**

1. Hetrocyclische Amidoxim-Derivate, die der Formel (I)

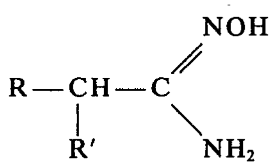

(I)

entsprechen, in welcher

R' ein Wasserstoffatom oder den Methylrest und
R den 2-(1-Oxo-1H-isochinoleinyl)- oder dem 2-(1-Oxo-1,3-dihydro-2H-isoindolyl)-Rest

bedeutet, und deren Säureadditionssalze mit pharmazeutisch annehmbaren Säuren sowie Racemate und Enantiomere der Verbindungen, in welchen R' $CH_3$ bedeutet.

2. Derivate nach Anspruch 1, in welchen R' $CH_3$ bedeutet.
3. $\alpha$-Methyl-2-(1-Oxo-1H-isochinolein)-acetamidoxim.
4. 2-(1-Oxo-1H-isochinolein)-acetamidoxim.
5. Verfahren zur Herstellung der Verbindungen nach Anspruch 1, dadurch gekennzeichnet, daß man eine Verbindung RH mit einem Nitril

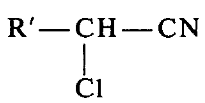

in Gegenwart von Natriumhydrid umsetzt und dann das erhaltene Nitril

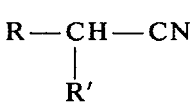

mit Hydroxylaminchlorhydrat zur Reaktion bringt.

6. Heilmittel, dadurch gekennzeichnet, daß es eine in irgend einem der Ansprüche 1 bis 4 dargelegten Verbindung enthält.

7. Pharmazeutische Zusammensetzung, dadurch gekennzeichnet, daß sie eine in irgend einem der Ansprüche 1 bis 4 dargelegte Verbindung in Kombination mit irgend einem geeigneten Träger enthält.

**Patentanspruch für den Vertragsstaat: AT**

Verfahren zur Herstellung von heterocyclischen Amidoxim-Derivaten, die der Formel (I)

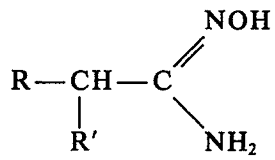

(I)

entsprechen, in welcher

R' ein Wasserstoffatom oder den Methylrest und
R den 2-(1-Oxo-1H-isochinoleinyl)- oder den 2-(1-Oxo-1,3-dihydro-2H-isoindolyl)-Rest

darstellt, sowie ihrer Säureadditionssalze mit pharmazeutisch annehmbaren Säuren und der Racemate und Enantiomeren der Verbindungen, in welchen R' für $CH_3$ steht, dadurch gekennzeichnet, daß man eine Verbindung RH in Gegenwart einer Base, wie Natriumhydrid, mit einem Nitril

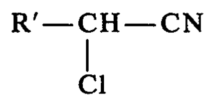

umsetzt und dann das erhaltene Nitril

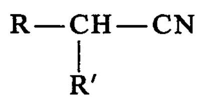

mit Hydroxylaminchlorhydrat zur Reaktion bringt.

**Claims for the contracting states: BE, CH, DE, FR, GB, IT, LI, LU, NL, SE**

1. Heterocyclic amidoxime derivatives responding to formula (I)

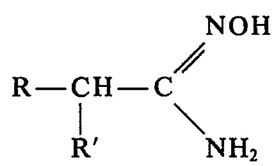

(I)

wherein

R' represents a hydrogen atom or the methyl group, and
R represents the 1-oxo-1H-isoquinolin-2-yl or dihydro-1,3-oxo-1-2H-isoindol-2-yl group,

and their addition salts with pharmaceutically acceptable acids, and the racemantes and enantiomers of the compounds wherein R' is $CH_3$.

2. Compounds according to claim 1 wherein R' is $CH_3$.

3. $\alpha$-Methyl-1-oxo-1H-isoquinoline-2-acetamidoxime.

4. Oxo-1-1H-isoquinoline-2-acetamidoxime.

5. A process for the preparation of compounds according to claim 1, characterized in that a compound RH is reacted with a nitrile

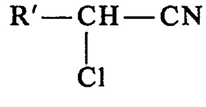

in the presence of sodium hydride, and then the obtained nitrile

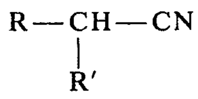

is reacted with hydroxylamine hydrochloride.

6. A Medicament characterized in that it contains a compound as specified in any one of claims 1 to 4.

7. A pharmaceutical composition, characterized in that it contains a compound as specified in any one of claims 1 to 4 in association with any suitable excipient.

**Claims for the contracting state: AT**

A process for the preparation of heterocyclic amidoxime derivatives responding to formula (I)

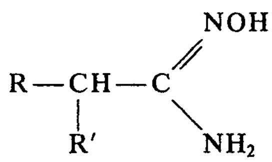

(I)

wherein

R′ represents a hydrogenatom or the methyl group, and
R represents the 1-oxo-1H-isoquinolin-2-yl or dihydro-1,3-oxo-1-2H-isoindol-2-yl group,

and their addition salts with pharmaceutically acceptable acids, and the racemates and enantiomers of the compounds wherein R′ is $CH_3$, process chararacterized in that a compound RH is reacted with a nitrile

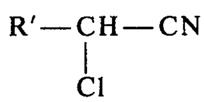

in the presence of a base such as sodium hydride, and then the obtained nitrile

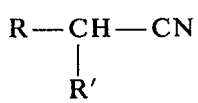

is reacted with hidroxylamine hydrochloride.